(19) Europäisches Patentamt / European Patent Office / Office européen des brevets

(11) **EP 4 296 653 A1**

(12) **EUROPEAN PATENT APPLICATION**
published in accordance with Art. 153(4) EPC

(43) Date of publication:
**27.12.2023 Bulletin 2023/52**

(21) Application number: **22775014.8**

(22) Date of filing: **04.03.2022**

(51) International Patent Classification (IPC):
**G01N 21/64** (2006.01)   **C12M 1/34** (2006.01)
**C12Q 1/70** (2006.01)   **G01N 21/03** (2006.01)
**G01N 21/78** (2006.01)   **G01N 33/483** (2006.01)

(52) Cooperative Patent Classification (CPC):
**C12M 1/34; C12Q 1/70; G01N 21/03; G01N 21/64; G01N 21/78; G01N 33/483**

(86) International application number:
**PCT/JP2022/009369**

(87) International publication number:
**WO 2022/202218 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2021   JP 2021053011**

(71) Applicant: **National Institute Of Advanced Industrial Science and Technology Chiyoda-ku Tokyo 100-8921 (JP)**

(72) Inventors:
• **FUJIMAKI Makoto**
  **Tsukuba-shi, Ibaraki 305-8560 (JP)**
• **ASHIBA Hiroki**
  **Tsukuba-shi, Ibaraki 305-8560 (JP)**

(74) Representative: **Gille Hrabal Partnerschaftsgesellschaft mbB Patentanwälte Brucknerstraße 20 40593 Düsseldorf (DE)**

(54) **BIOLOGICAL SUBSTANCE DETECTION METHOD USING WELL ARRAY AND PARTICLES, WELL ARRAY, AND DETECTION DEVICE**

(57)   To detect a biological substance at high speed and high sensitivity by using a well array and particles. The biological substance detection method of the present invention includes a step of preparing a test liquid containing particles at a concentration of at least $5 \times 10^7$ particles/mL, a step of allowing the particles to trap the biological substance to form a trapped body, a step of sending the test liquid onto the well array to store a plurality of the particles including the trapped body in the wells while storing the number of the particles to be stored in one well to a minimum average storage number $N_{min}$ represented by the following formulas (1) and (2), and a step of detecting the color development of the well with an image pickup element.

**(Cont. next page)**

[Mathematical Formula 1]

$$N_{min} = C \times V_L \times \frac{P_{image}}{P_{total}} \qquad (1)$$

$$N_{min} \geqq 8 \qquad (2)$$

In the formula (1), C represents a concentration of the particles in the test liquid; $V_L$ represents an amount of the test liquid and is at least 5 $\mu$L; $P_{total}$ represents the number of pixels of the image pickup element and is at least 300,000 pixels; and $P_{image}$ represents the number of pixels of a pixel group used for imaging one of the wells in the image pickup element and is at least 9 pixels.

## FIG.2(a)

1,000x 10.0μm WD:17.4mm 15kV 2021/01/22

**EP 4 296 653 A1**

**Description**

Technical Field

The present invention relates to a biological substance detection method using a detection system having a detection element whose size is set for high-speed detection of a biological substance, a well array, and a detection device.

Background Art

As a protein detection method, a digital ELISA method (Enzyme-Linked Immuno Sorbent Assay) is known (refer to Non-Patent Documents 1 to 5, and Patent Documents 1 to 6), wherein, regarding a magnetic bead group which may include magnetic beads which have not trapped the protein and magnetic beads which have trapped the protein to which a labeled molecule having an enzyme is to be bound, the respective magnetic beads are moved and stored in microwells of a microwell array having a large number of microwells arranged on a substrate, respectively, by using a magnet or gravity settling, and digitally counts the number of the microwells which have developed color. It is also reported that a virus-derived protein is detected using the digital ELISA method (refer to Non-Patent Document 6).

The microwell contains therein a fluorescent substrate and when the magnetic bead contained in the microwell has trapped the protein, that is, the protein is present in the microwell, color development (light emission) occurs due to an enzyme reaction between the enzyme and the fluorescent substrate and the presence or absence of the protein is observed as presence or absence of the color development of the microwell. At the same time, by digitally counting the number of the microwells causing color development (for example, by counting the microwell causing color development as "1" and the microwell not causing color development as "0"), the number of the virus in a sample can be detected and highly quantitative protein detection can be realized. When the present method is used for the detection of a virus protein as Non-Patent Document 6, highly quantitative virus detection can be realized.

In the digital ELISA method, however, one of the magnetic beads is stored in one of the microwells in order to secure quantitativity so that the number of the magnetic beads which can be introduced in a test liquid is limited by the formation number of the microwells and the test liquid inevitably contains therein the magnetic beads at a low concentration (content). As a result, contact opportunities between the magnetic beads and the protein in the test liquid are limited and it takes a lot of reaction time for the magnetic beads to trap the protein. On the other hand, even if shortening of the reaction time is tried by increasing the concentration of the magnetic beads in the test liquid, the number of the magnetic beads exceeds the formation number of the microwells. Then, some of the magnetic beads cannot be stored in the microwells and the protein trapped by such magnetic beads is omitted from the detection, leading to deterioration in detection sensitivity.

In order to avoid this detection omission of a virus due to these magnetic beads which cannot be completely stored in the microwells, a simple attempt to increase the formation number of the microwells in the microwell array has also been reported (refer to Patent Documents 1 and 3).

However, with an increase in the formation number of the microwells, an observation area becomes wider and at the same time, time necessary for observation becomes longer.

This means that a simple increase in the formation number of the microwells is not enough for achieving the detection of a biological substance such as virus in a short time because the observation time becomes longer.

Prior Art Documents

Patent Documents

Patent Document 1: International Publication No. 2016/6208
Patent Document 2: Japanese Patent Laid-Open No. 2018-91737
Patent Document 3: International Publication No. 2012/121310
Patent Document 4: Japanese Patent No. 3886161
Patent Document 5: Japanese Patent No. 5551798
Patent Document 6: Japanese Patent No. 5363663

Non-Patent Documents

Non-Patent Document 1: David M Rissin, et al., Nature Biotechnology Vol. 28, No. 6, pp.595(2010)
Non-Patent Document 2: Elena Perez-Ruiz, et al., Analytica Chimica Acta 1015, pp.74(2018)
Non-Patent Document 3: Soo Hyeon Kim, et al., Lab on a Chip 12, pp.4986(2012)
Non-Patent Document 4: David M Rissin, et al., Analytical Chemistry 83, pp.2279(2011)
Non-Patent Document 5: Stephanie M. Schubert, et al., Analytical Chemistry 88, pp.2952(2016)
Non-Patent Document 6: Karen Leirs, et al, Analytical Chemistry 88, pp.8450(2016)

Disclosure of the Invention

Problems to be solved by the Invention

An object of the present invention is to overcome the various problems of the prior art and provide a biological substance detection method, a well array, and a detection device capable of detecting a biological substance at high speed and high sensitivity by using the well array and particles.

In the prior art, a factor that prevents shortening of a detection time of the biological substance is storing one bead in one well.

Although this method contributes to quantification of the biological substance to be detected, it therefore cannot respond to the social need that prioritizes high-speed detection in a test for infection of a virus or the like.

The present inventors have therefore decided to newly search for a biological substance detection method specialized in high-speed detection.

The policy of the prior art is changed and storing a plurality of particles (beads) in one well is studied. Then, the number of the particles to be stored in one well will be an issue to be solved.

Assuming that in the detection of pathogenic viruses having a diameter of about 100 nm such as an influenza virus or a corona virus, the particles bind to the pathogenic viruses when they collide to each other in a liquid, in other words, a reaction probability is 100%, and a test liquid containing the particles at a concentration of $5 \times 10^7$ particles/mL is used, a reaction time necessary for the particles having a particle size of 1 $\mu$m to trap half of the pathogenic viruses is about 500 seconds, for the particles having a particle size of 2 $\mu$m to trap them is about 240 seconds, and for the particles having a particle size of 3 $\mu$m to trap them is about 150 seconds. It is to be noted that the reaction between the pathogenic viruses serving as antigens and the particles is an antigen-antibody reaction in the particles having a spherical form and a large number of antibodies that specifically bind to the antigens and are bound onto the spherical surface of the particles. Here, the binding capacity (dissociation constant $K_D$) of the antibody to the pathogenic viruses is estimated at 50 nM in consideration of the performance of a generally available antibody.

The reaction time between the pathogenic viruses and the particles at the concentration of the particles in the test liquid increased to $1 \times 10^8$ particles/mL is about 210 seconds when the particle size of the particles is 1 $\mu$m, about 110 seconds when the particle size of the particles is 2 $\mu$m, and about 70 seconds when the particle size of the particles is 3 $\mu$m. The reaction time between the pathogenic viruses and the particles at the concentration of the particles in the test liquid increased to $5 \times 10^8$ particles/mL is about 110 seconds when the particle size of the particles is 0.5 $\mu$m, about 65 seconds when the particle size of the particles is 1 $\mu$m, about 35 seconds when the particle size of the particles is 2 $\mu$m, and about 15 seconds when the particle size of the particles is 3 $\mu$m.

From these results, it has been confirmed that with an increase in the concentration of the particles in the test liquid and with an increase in the particle size of the particles, the reaction time tends to be shorter. In addition, when a substance to be detected is smaller such as a protein, the reaction time is shorter.

Various attempts have been made in addition by changing the concentration and particle size of the particles, but considering the length of the reaction time, the concentration of the particles is required to be at least $5 \times 10^7$ particles/mL.

As a detection device to be used for the detection of the biological substance, a general-purpose observation device comprised of an image pickup element having 300,000 pixels with an estimated VGA image pickup element (640 $\times$ 480 pixels) is used as a baseline. The most standard image pickup element at present is supposed to be a full HD image pickup element. However, when a plurality of the particles are stored in one of the wells, compared with the case where one of the particles is stored in one of the wells as in the prior art, the number of the wells necessary for observing the same number of the particles is less. And then, the VGA image pickup element less expensive than the full HD image pickup element is set as a base line. There is considered the use of a higher-resolution image pickup element (full HD image pickup element, 4K image pickup element, 8K image pickup element or the like) than the VGA image pickup element, but in order to establish a general-purpose detection method that can respond to even the occurrence of a large number of patients infected with a virus, the standard and less expensive general-purpose observation device is used as a baseline.

The lower limit of the amount of the test liquid is estimated at 5 $\mu$L because a virus detection test can be carried out easily at such an amount. The liquid amount of 5 $\mu$L is an amount easy to handle with a commonly-used micropipette.

For imaging of one of the wells, 3 × 3 pixels (= 9 pixels) are necessary at minimum. It is therefore necessary to determine, as the baseline, a total number of the wells to be formed in the well array at about 33,333 (300,000 pixels/9 pixels).

In order to store, in the wells of the above well array, all the particles contained in 5 μL of the test solution having a particle concentration of 5 × 10⁷ particles/mL, 8 or more particles on average are required to be stored in one of the wells [(5 × 10⁷ particles/mL × 5 μL/33,333 wells) = 7.5 particles].

The aforesaid 8 particles, that is, the average number of the particles to be stored in one of the wells, are the minimum average storage number of particles under the conditions where the concentration of the particles is thinnest. For the use of the test liquid prepared by increasing the concentration of the particles, one of the wells is formed by setting the capacity while increasing the storage number.

As an amount of the test liquid, 100 μL, that is, an amount ordinarily used in PCR test or the like, or more (for example, 1 mL) may be considered for the detection use. As the amount of the test liquid is larger, the total number of the particles contained in the test liquid increases so that one of the wells is formed by setting the capacity while increasing the storage number.

Also, as described above, a high-performance observation device having a high-resolution image pickup element (full HD image pickup element, 4K image pickup element, or 8K image pickup element) may be considered for use.

In addition, the number of pixels in a pixel group used for imaging one of the wells may be 4 × 4 pixels (= 16 pixels) or more to improve the visibility of the well.

By the way, with the aforesaid high-performance observation device, the number of pixels of the image pickup element increases and therefore, the total number of the wells formed in the well array can be increased to more than the baseline number 33,333. As a result, the number of the particles to be stored in one of the wells may be made smaller than 8 on average. However, in addition to the high cost of the high-performance observation device itself, the total number of the wells is too large relative to the total number of the particles to be contained in the test liquid estimated from the concentration of the particles in the test liquid or the amount of the test liquid in the case where the total number of the wells is increased depending on the number of pixels. This makes it difficult to prepare the well array and makes the cost needlessly high. Further, since the number of the particles to be stored in one well is less than 8 on average, individual wells should be prepared to have a smaller size, which inevitably increases the preparation cost of the well array. Still further, even if the number of pixels and the total number of the wells are increased needlessly, there occurs a case where the number of the particles to be stored in one of the wells becomes zero, which only disturbs efficient virus detection.

The high-performance observation device is therefore used only in the case where the concentration of the particles in the test liquid is high or the amount of the test liquid is large, in other words, where the total number of the particles contained in the test liquid is large. The minimum average storage number of the particles to be stored in one of the wells is therefore determined to 8 as estimated from the baseline.

In these meanings, the minimum average storage number $N_{min}$ of the particles to be stored in one of the wells may satisfy the following formulas (1) and (2):

[Mathematical Formula 1]

$$N_{min} = C \times V_L \times \frac{P_{image}}{P_{total}} \quad (1)$$

$$N_{min} \geqq 8 \quad (2)$$

wherein, in the formula (1), C represents the concentration of the particles in the test liquid and is at least 5 × 10⁷ particles/mL; $V_L$ represents the amount of the test liquid and is at least 5 μL; $P_{total}$ represents the number of pixels of the image pickup element and is at least 300,000 pixels; and $P_{image}$ represents the number of pixels of a pixel group used for imaging one of the wells in the image pickup element and is at least 9 pixels (3 × 3 pixels).

As a result, it has been found that by setting the number of the particles to be stored in one of the wells to at least $N_{min}$ (particles) on average, it is possible to store all the particles in the well, keep a detection sensitivity, and shorten the reaction time, and thereby detect the biological substance at high speed.

Means for solving the Problems

The present invention is based on the aforesaid finding and the following are means for solving the aforesaid problem.

<1> A biological substance detection method using a well array formed by defining a plurality of wells adjacent to each other with a side wall stood on a substrate and particles capable of trapping a biological substance and detecting the biological substance in a test liquid based on color development detection of the wells, including a test liquid preparation step for preparing the test liquid containing the particles at a concentration of at least $5 \times 10^7$ particles/mL, a biological substance trapping step for allowing the particles to trap the biological substance to form a trapped body, a particle storing step for sending the test liquid onto the well array to store a plurality of the particles including the trapped body in the wells while storing the number of the particles to be stored into one of the wells to at least a minimum average storage number $N_{min}$ represented by the following formulas (1) and (2), and a color development detection step for detecting color development of the wells with an image pickup element having at least 300,000 pixels.

[Mathematical Formula 2]

$$N_{min} = C \times V_L \times \frac{P_{image}}{P_{total}} \qquad (1)$$

$$N_{min} \geqq 8 \qquad (2)$$

wherein, in the formula (1), C represents the concentration of the particles in the test liquid and is at least $5 \times 10^7$ particles/mL; $V_L$ represents the amount of the test liquid and is at least 5 $\mu$L; $P_{total}$ represents the number of pixels of the image pickup element and is at least 300,000 pixels; and $P_{image}$ represents the number of pixels of a pixel group used for imaging one of the wells in the image pickup element and is at least 9 pixels ($3 \times 3$ pixels).

<2>The biological substance detection method as described above in <1>, wherein the particles are magnetic particles having a diameter of 0.1 $\mu$m to 6 $\mu$m.

<3> The biological substance detection method as described above in <1> or <2>, wherein the test liquid preparation step is to prepare the test liquid while adjusting the concentration of the particles to $5 \times 10^7$ particles/mL to $5 \times 10^9$ particles/mL.

<4> The biological substance detection method as described above in any of <1> to <3>, wherein the wells each have a volume of 2 fL to 100 pL.

<5> The biological substance detection method as described above in any of <1> to <4>, wherein the color development detection step is carried out by fixing an observation visual field of the image pickup element to have a size including a well formation area which is an area of the substrate in a well formation region.

<6> The biological substance detection method as described above in any of <1> to <5>, wherein color development of the wells in the color development detection step is due to a reaction between the biological substance of the trapped body and a color producing reagent causing the color development of the wells.

<7> A well array for use in the biological substance detection method as described above in any of <1> to <6>, which can store a plurality of the particles including the trapped body in one of the wells while storing the number of the particles to at least a minimum average storage number $N_{min}$ represented by the following formulas (1) and (2):

[Mathematical Formula 3]

$$N_{min} = C \times V_L \times \frac{P_{image}}{P_{total}} \quad (1)$$

$$N_{min} \geqq 8 \quad (2)$$

wherein, in the formula (1), C represents the concentration of the particles in the test liquid and is at least $5 \times 10^7$ particles/mL; $V_L$ represents the amount of the test liquid and is at least 5 μL; $P_{total}$ represents the number of pixels of the image pickup element and is at least 1300,000 pixels; and $P_{image}$ represents the number of pixels of a pixel group used for imaging one of the wells in the image pickup element and is at least 9 pixels ($3 \times 3$ pixels).

<8> A detection device including a detection chip having the well array as described above in <7> and a detection unit having an image pickup element having a pixel number of at least 300,000 pixels.

Advantageous Effects of the Invention

The present invention makes it possible to solve the aforesaid various problems of the prior art and provide a biological substance detection method, a well array, and a detection device capable of detecting a biological substance at high speed and high sensitivity by using the well array and particles.

Brief Description of the Drawings

FIG. 1 is an explanatory drawing for explaining the setting of wells.
FIG. 2(a) is an electron microscope image in which Preparation Example 1 of a well array has been imaged.
FIG. 2(b) is a partially enlarged picture of FIG. 2(a).
FIG. 3(a) shows an electron microscope image in which Preparation Example 2 of a well array has been imaged.
FIG. 3(b) is a partially enlarged picture of FIG. 3(a).
FIG. 4 is a drawing showing an embodiment of a detection device.
FIG. 5(a) is an explanatory drawing (1) for explaining the detection manner of a biological substance.
FIG. 5(b) is an explanatory drawing (2) for explaining the detection manner of a biological substance.
FIG. 5(c) is an explanatory drawing (3) for explaining the detection manner of a biological substance.
FIG. 6 is a graph showing the results of Example of the biological substance detection according to the present invention.

Mode for carrying out the Invention

[Biological substance detection method]

The biological substance detection method of the present invention is to use a well array formed by defining a plurality of wells adjacent to each other with a side wall stood on a substrate and particles capable of trapping a biological substance, and to detect the biological substance in a test liquid based on the detection of color development of the wells, which includes a test liquid preparation step, a biological substance trapping step, a particle storing step, and a color development detection step.

The biological substance is not particularly limited and examples include DNA, RNA, proteins, viruses, and bacteria to be detected by a known biological substance detection method such as an ELISA method and an immunoassay method. In particular, the examples include biological substances having a diameter of 1 nm to 500 nm such as protein (having a diameter of about 5 nm) and pathogenic viruses such as an influenza virus and a corona virus (having a diameter of about 100 nm).

A liquid sample containing the biological substance is not particularly limited and examples include blood, saliva, urea, and environmental water.

<Test liquid preparation step>

The test liquid preparation step is to prepare the test liquid by setting the concentration of the particles to at least $5 \times 10^7$ particles/mL.
For example, for the detection of a virus contained in the saliva, the test liquid is prepared by adding the particles to the saliva.
A method of setting a specific concentration of the particles in the test liquid is not particularly limited and examples include a method of diluting a known particle-containing liquid containing the particles at a high concentration with a known buffer solution.
The particles are not particularly limited and examples include known plastic particles, metal particles, ceramic particles, and magnetic particles. The magnetic particles are preferred, because they can be stored in the wells more easily using a magnet, in comparison with the plastic particles, metal particles, and ceramic particles that precipitate in the wells by their own weight.
The particle size of the particles is not particularly limited and is preferably 0.1 $\mu$m to 9 $\mu$m and more preferably 0.2 $\mu$m to 5 $\mu$m.
As the particles, spherical ones and also those having a plurality of particle sizes such as oval ones can be used. When the particles are oval, the maximum diameter corresponds to the particle size described above in the particle size range.
The particle size of the particles will hereinafter be described in detail.
When the particle size is too small, even if the magnetic particles are used, they have low responsiveness to magnetic force so that it sometimes takes a lot of time to perform an operation of attracting the particles by a magnet and storing them in the well.
The lower limit of the particles size is therefore preferably 0.1 $\mu$m or more and more preferably 0.2 $\mu$m or more.
On the other hand, using the particles having a larger particle size can shorten the reaction time with the biological substance, but too large particles require a large observation visual field and may prolong the detection time during color development detection of the wells, which will hereinafter be described in detail.
The depth of the well is preferably 40 $\mu$m at most. The well deeper than this depth is likely to cause such inconveniences that the image pickup element loses its focus in the whole depth direction of the well, it becomes difficult to process the well, and the test liquid cannot easily be stored in the well.
When preparing 5 $\mu$L of the test liquid having the particle concentration of $5 \times 10^7$ particles/mL, the total number of the particles contained in the test liquid is $2.5 \times 10^5$ particles.
The particles even in the spherical form are not closest-packed in the wells so that, similarly to another form, they are assumed to be packed in a cubic lattice form.
In this assumption, when the particles have a particle size of 1 $\mu$m, the total volume (capacity) of the well array obtained by integrating the volumes of all the wells is required to be $2.5 \times 10^5$ $\mu$m$^3$ (1 $\mu$m $\times$ 1 $\mu$m $\times$ 1 $\mu$m $\times$ $2.5 \times 10^5$ particles). Similarly, when the particles have a particle size of 5 $\mu$m, the total volume is required to be $3.13 \times 10^7$ $\mu$m$^3$, and when the particles have a particle size of 6 $\mu$m, the total volume is required to be $5.4 \times 10^7$ $\mu$m$^3$.
Supposing that the well has a depth of 40 $\mu$m or less, a total opening area of the well array obtained by integrating the opening area of all the wells (an area obtained by integrating the formation area of all the wells on the substrate of the well array) is required to be 0.00625 mm$^2$ or more ($2.5 \times 10^5$ $\mu$m$^3$/40 $\mu$m or less) when the particles have a particle size of 1 $\mu$m. Similarly, the total opening area is required to be 0.169 mm$^2$ or more when the particles have a particle size of 3 $\mu$m; 0.4 mm$^2$ or more when the particles have a particle size of 4 $\mu$m; 0.781 mm$^2$ or more when the particles have a particle size of 5 $\mu$m; 1.35 mm$^2$ or more when the particles have a particle size of 6 $\mu$m; 2.14 mm$^2$ or more when the particles have a particle size of 7 $\mu$m; 3.2 mm$^2$ or more when the particles have a particle size of 8 $\mu$m; and 4.56 mm$^2$ or more when the particles have a particle size of 9 $\mu$m; and 6.25 mm$^2$ or more when the particles have a particle size of 10 $\mu$m.
Assuming that the color development detection of the wells is performed at the detection unit comprised of the general-purpose observation device, an observation visual field is about 5 mm$^2$ when a 4$\times$ objective lens is used in the general-purpose observation device and it is about 0.8 mm$^2$ when a 10$\times$ objective lens is used. As the observation visual field is smaller, the visibility of the wells is higher and the color development detection is easier.
When the total opening area of the well array exceeds the observation visual field, the area exceeding the observation visual field is required to be observed by moving the observation visual field. It therefore takes time for detecting the color development of the wells.
Consequently, the upper limit of the particle size is preferably 9 $\mu$m or less under the conditions where the total opening area does not exceed the observation visual field at the observation visual field of 5 mm$^2$ and it is more preferably 5 $\mu$m or less under the conditions where the total opening area does not exceed the observation visual field at the observation visual field of 0.8 mm$^2$.

The lower limit of the concentration of the particles in the test liquid may be $5\times10^7$ particles/mL or more and is more preferably $1\times10^8$ particles/mL or more because with an increase in the concentration, the reaction time with the biological substance can be shortened.

When the concentration is too high, it may be over performance for shortening of the reaction time. In addition, it may increase the observation visual field and prolong the detection time during the color development detection of the wells. The following is the details thereof.

When the particles have a particle size of 1 $\mu$m on the same assumption as when finding the preferable particle size (the amount of the test liquid is 5 $\mu$L and the depth of the well is 40 $\mu$m or less), the total volume is $5 \times 10^6$ $\mu$m$^3$ and the total opening area is 0.125 mm$^2$ or more at the concentration of $1 \times 10^9$ particles/mL; the total volume is $1 \times 10^7$ $\mu$m$^3$ and the total opening area is 0.25 mm$^2$ or more at the concentration of $2 \times 10^9$ particles/mL; the total volume is $2 \times 10^7$ $\mu$m$^3$ and the total opening area is 0.50 mm$^2$ or more at the concentration of $4 \times 10^9$ particles/mL; the total volume is $4 \times 10^7$ $\mu$m$^3$ and the total opening area is 1.0 mm$^2$ or more at the concentration of $8 \times 10^9$ particles/mL; the total volume is $8 \times 10^7$ $\mu$m$^3$ and the total opening area is 2.0 mm$^2$ or more at the concentration of $1.6 \times 10^{10}$ particles/mL; and the total volume is $2.0 \times 10^8$ $\mu$m$^3$ and the total opening area is 5.0 mm$^2$ or more at the concentration of $4.0 \times 10^{10}$ particles/mL. The visual field to be observed in practice has an area obtained by adding, to the total opening area, the thickness of the side wall, that is, the area of the side wall portion so that at the concentration of $4.0 \times 10^{10}$ particles/mL, an observation visual field more than 5.0 mm$^2$ becomes necessary in practice. This means that at the concentration more than $4.0 \times 10^{10}$ particles/mL, the total opening area is larger relative to the observation visual field (5 mm$^2$).

Thus, an excessively high concentration may be a limitation for setting the total opening area of the well array to be the observation visual field (5 mm$^2$) or less.

This limitation is relaxed as the particle size of the particles is smaller (for example, 0.1 pm), but an unnecessary increase in the number of the particles is wasteful.

Accordingly, the upper limit of the concentration is preferably $4 \times 10^{10}$ particles /mL or less based on the balance between the detection time and the reaction time, and is more preferably $5 \times 10^9$ particles/mL or less in consideration of the thickness of the side wall.

<Biological substance trapping step>

The biological substance trapping step is to allow the particles to trap the biological substance to form a trapped body. The trapped body is formed by binding the biological substance to the particles.

A mode of allowing the particles to trap the biological substance is not particularly limited and can be selected as needed depending on the purpose. Examples thereof include an antigen-antibody reaction, DNA hybridization, biotin-avidin binding, and amino binding. For example, in the antigen-antibody reaction, the particles with a large number of antibodies, which specifically bind to the biological substance used as an antigen, bound to their surfaces are used.

As the particles capable of trapping the biological substance, commercially available ones may be used, or they may be formed by a known method.

In the test liquid containing the particles at a low concentration, contact opportunities between the biological substance and the particles are limited and the reaction time is longer. The test liquid preparation step is therefore performed as a preliminary step of the biological substance trapping step to obtain the test liquid having an increased particle concentration.

Examples of a method of performing the biological substance trapping step include a method of stirring the test liquid to bring the particles into contact with the biological substance.

<Particle storing step>

The particle storing step is to send the test liquid onto the well array to store, in one of the wells, a plurality of the particles including the trapped body while storing the number of the particles to at least a minimum average storage number $N_{min}$ represented by the following formulas (1) and (2).

[Mathematical Formula 4]

$$N_{min} = C \times V_L \times \frac{P_{image}}{P_{total}} \qquad (1)$$

$$N_{min} \geqq 8 \qquad (2)$$

wherein, in the formula (1), C represents the concentration of the particles in the test liquid and is at least $5 \times 10^7$ particles/mL; $V_L$ represents an amount of the test liquid and is at least 5 $\mu$L; $P_{total}$ represents the number of pixels of the image pickup element and is at least 300,000 pixels; and $P_{image}$ represents the number of pixels of a pixel group used for imaging one of the wells in the image pickup element and is at least 9 pixels ($3 \times 3$ pixels).

As examples of a larger number of pixels of the image pickup element, there are 2,073,600 pixels (1,920 $\times$ 1,080 pixels) for a full HD image pickup element, 8,294,400 pixels (3,840 $\times$ 2,160 pixels) for a 4K image pickup element, 33,177,600 pixels (7,680 $\times$ 4,320 pixels) for an 8K image pickup element, and about 61,000,000 pixels for a full-size CMOS image sensor.

The pixel group means a pixel group which consists of pixels juxtaposed in a first direction and pixels juxtaposed in a second direction orthogonal to the first direction and in which these pixels are arranged in matrix form. In a pixel group arranged in a matrix of 3 rows and 3 columns, that is, a group of $3 \times 3$ pixels (9 pixels), one pixel at the center is used for the color development detection of the wells. The pixel group may be comprised of $4 \times 4$ pixels (16 pixels) or the like and in this case, the number of pixels used for the color development detection of the wells can be increased and this leads to improvement in visibility.

The term "average" in the minimum average storage number $N_{min}$ is for taking into account the variation during storing the particles in the well and the individual wells themselves are set to be able to contain at least $N_{min}$ particles. Whether or not at least $N_{min}$ particles are stored in the well is confirmed by "average value" in consideration of the variation. For example, it is confirmed from "average value" obtained by counting the total number of the particles stored in the wells in the arbitrarily selected group of the wells (for example, four wells arranged in two rows and two columns and adjacent to each other) from an electron microscope image or optical microscope image and then dividing the total number by the number of the wells in the group of the wells to be counted.

-Well Array-

The well array is formed by defining a plurality of the wells adjacent to each other by the side wall stood on a substrate.

The well array is comprised so that one of the wells can contain a plurality of the particles including the trapped body with at least the minimum average storage number $N_{min}$ represented by the aforesaid formulas (1) and (2).

The total number of the wells to be formed in the well array is not particularly limited but ideally it is equal to an observation limit $P_{total}/P_{image}$. The observation limit $P_{total}/P_{image}$ will next be described specifically. Supposing that the $P_{total}$ is 640 $\times$ 480 pixels (= 307,200 pixels) of the VGA image pickup element and the $P_{image}$ is 3 $\times$ 3 pixels (= 9 pixels), the observation limit $P_{total}/P_{image}$ is about 34,080 wells (213 $\times$ 160 wells). If the pixel number of the VGA image pickup element is more plainly 300,000 pixels, the observation limit is 33,333 wells.

A preferable lower limit of the total number of the wells will next be examined.

The premise is that even if the total number of the wells is less than $P_{total}/P_{image}$, the volume of one of the wells may be set so that the storage number of the particles may exceed the minimum average storage number $N_{min}$.

This means that the assumption that the total number of the wells is set at about 33,333 (300,000 pixels/9 pixels) is a baseline for setting the minimum average storage number at 8 on average. The total number of the wells can arbitrarily be reduced from the aforesaid assumption by making the storage number larger than the average value of 8 through volume setting of one of the wells.

However, the actual volume of the well has an upper limit because of the reason described later and the preferable maximum volume of the well is 100 pL (=0.1 $\times$ 10^6 $\mu$m^3).

On the other hand, when the preferably usable particles having a particle size of 1 $\mu$m are used, the total volume of the well array is required to be $2.5 \times 10^5$ $\mu$m^3 or more to store, in the wells, all of $2.5 \times 10^5$ particles (the total number of particles when 5 $\mu$L of the test liquid having a particle concentration of $5 \times 10^7$ particles/mL is prepared).

Therefore, the lower limit of the total number of the wells could be preferably 3 ($2.5 \times 10^5$ $\mu$m$^3$/ ($0.1 \times 10^6$ $\mu$m$^3$)) or more.

In an actual detection case, on the other hand, even when a detection object is the test liquid not containing the biological substance, a certain number of color-developed wells (false-positive wells) are detected due to a detection reagent being unwashed or the like. The present inventors repeated a preliminary test using enzymes, including $\beta$-galactosidase, which cause color development of a well 1, and confirmed the occurrence of about tens of the false-positive wells at most.

When carrying out detection of the test liquid containing the biological substance, therefore, in order to detect the number of color-developed wells as a significant signal derived from the biological substance, it is required to take into account the variation and set the number of the wells so that color-developed wells as significantly more than the false-positive wells (for example, $\mu + 3.3\sigma$ wells or more in which $\mu$ represents an average number of the false-positive wells and $\sigma$ represents a standard deviation) can be detected. From this viewpoint, the lower limit of the total number of the wells is preferably 100 or more.

By the way, in the biological substance detection method of the present invention, quantitativity according to the Poisson distribution is secured in terms of the statistical probability insofar as the total number of the wells exceeds the number of the biological substance contained in the test liquid.

In addition, with an increase in the total number of the wells used for detection, a dynamic range in digital counting of the number of color-developed wells expands, which is remarkably advantageous in performing high-speed and high-sensitivity quantitative detection of the biological substance.

In order to add the quantitative detection of the biological substance to the object, how to set the lower limit of the total number of the wells after satisfying the aforesaid requirement for the lower limit depends on the number of the biological substance contained in the test liquid, that is, the intended use to which the biological substance detection method of the present invention is applied. For example, empirically, for use when the test liquid assumed to contain a small number of the biological substance is to be detected, the total number of the wells is set small (for example, 1,000 wells) so as to satisfy the setting of the dynamic range based on the number of the biological substance. On the contrary, for use when the test liquid assumed to contain a large number of the biological substance is to be detected, the total number of the wells is set large (for example 10,000 wells).

Regarding the upper limit of the total number of the wells, if the total number of the wells exceeds $P_{total}/P_{image}$, with the entirety of the well array being observed, the color development of the well cannot be detected due to deteriorated visibility. Also, if the observation visual field is divided to partially observe the well array, it takes time to detect the color development of the well because the observation should be carried out by transferring the observation visual field with an extra time.

In the well array, a well formation area which is an area of the substrate in the well formation region is preferably at least 0.8 mm$^2$. The well formation area indicates an area on the substrate on which a plurality of the wells are formed as a group of the wells to be observed and it is different from an area for the formation of one of the wells.

When the well formation area is 0.8 mm$^2$ and color development of the well is detected with the detection unit comprised of the general-purpose observation device having the 10$\times$ objective lens, observation can be carried out without transferring the observation visual field because the observation visual field in the detection unit is about 0.8 mm$^2$.

When the well formation area is 5 mm$^2$ and color development of the well is detected with the detection unit comprised of the general-purpose observation device having the 4$\times$ objective lens, observation can be carried out without transferring the observation visual field because the observation visual field in the detection unit is about 5 mm$^2$.

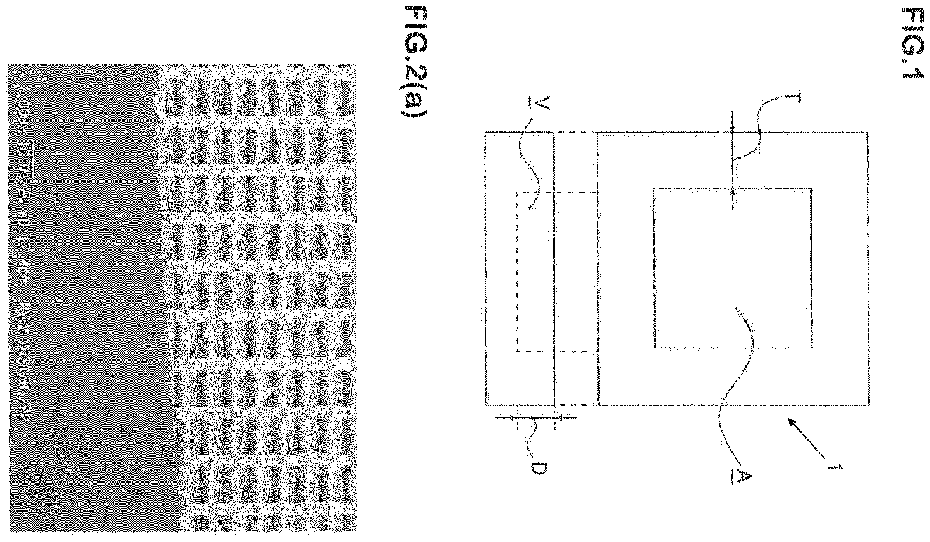

The constitution example of the well will next be described referring to FIG. 1. It is an explanatory drawing for explaining the setting of the well.

The depth D of the well 1 is not particularly limited and it is preferably 2 $\mu$m to 40 $\mu$m. The well 1 is designed for storing the particles therein so that the depth D is required to be larger than the particle size of the particles. The lower limit of the particle size of the usable particles is 0.1 $\mu$m so that if the depth D is less than 0.1 $\mu$m, even the particles having a minimum size are difficult to be stored in the well 1. The particles can be stably stored in the well having a deeper depth D so that even if particles having a small particle size are used, the depth is preferably set at 2 $\mu$m or more. On the other hand, the well having a depth D of more than 40 $\mu$m is likely to cause such inconveniences that the image pickup element loses its focus in the whole depth direction of the well, it becomes difficult to process the well, and the test liquid cannot easily be stored in the well.

The volume V of the well 1 is set on the baseline that the minimum average storage number $N_{min}$ of the particles are stored in the well.

Now consider that there are stored in the well 8 particles, that is, the minimum average storage number $N_{min}$ when the detection unit is comprised of the image pickup element having 300,000 pixels.

Supposing that the particles have a particle size of 0.1 $\mu$m, and the well 1 is not closest-packed with the particles and is designed with a margin to pack them in a cubic lattice form, the volume occupied by one of the particles in the well 1 is 0.001 fL. The volume occupied by 8 particles in the well is therefore 0.008 fL. The well 1 is therefore required to have a volume of at least 0.008 fL or more.

Given the visibility of the well 1 and the optimum value of the depth D, the length of one side of the well 1 smaller than 1 $\mu$m may deteriorate the visibility even in a high magnification microscopic observation system so that the length of one side is preferably 1 $\mu$m or more. As described above, the depth D is preferably 2 $\mu$m or more and therefore the lowest volume of the well satisfying such a size is 2 fL.

The lower limit of the volume V of the well 1 is therefore preferably 2 fL or more.

On the other hand, the upper limit of the volume V of the well 1 can be explained as follows.

When the volume V of the well 1 is excessively large, the concentration of a substance causing color development of the well 1 becomes lower in the well 1, causing deterioration in the visibility of the color development of the well 1. In other words, even when the biological-substance trapped body is stored in the well 1, the color development due to the biological substance in this well 1 cannot be distinguished from another well 1 storing only the particles which have not trapped the biological substance, making it difficult to detect the biological substance through color development or non-color development of the well 1.

On this point, according to a preliminary test performed by the present inventors with enzymes such as β-galactosidase that cause color development of the well 1, when there is one molecule of the enzymes in the well 1 filled with the test liquid and having a volume V of 10 pL, a color developed well 1 containing the enzyme can be distinguished from another well 1 not containing the enzyme and being in a non-color developed state.

About 100 molecules of the aforesaid enzyme can generally be attached to one virus via a substance, such as an antibody, that specifically adsorbs to a virus so that a detection limit between color development and non-color development of the well 1 can be estimated at 1,000 pL. If the volume of the well is set at 100 pL with a margin in the detection limit, color development and non-color development of the well 1 can easily be distinguished from each other.

Thus, the upper limit of the volume V of the well 1 is preferably 100 pL or less.

The side wall thickness T, which is a thickness of the side wall between the wells 1 adjacent to each other, is not particularly limited and it is preferably 0.5 $\mu$m to 15 $\mu$m. The side wall thickness T less than 0.5 $\mu$m makes processing of a well difficult and in addition such a well is easy to break. When the side wall thickness T is more than 15 $\mu$m, on the other hand, the well formation area in the well array becomes uselessly wide and a large limitation is likely to be imposed on the setting for the detection of the biological substance without transferring the observation visual field. When the distance between the wells 1 adjacent to each other is not uniform as in the case where the well 1 has a round or oblong shape, the side wall thickness T is determined by the thickness of the thinnest portion between the wells 1 adjacent to each other.

Although no limitation is imposed on the opening area A of the well 1, it is preferably 1 $\mu m^2$ to 50,000 $\mu m^2$ judging from the visibility and the relation (V/D) between depth D and volume V.

In an embodiment of the well 1 shown in FIG. 1, the opening shape is square, but the opening shape is not particularly limited. It may be a regular polygon such as a regular triangle and a regular hexagon (a honeycomb structure), a rectangle, a circle, or an ellipse. Also, the well 1 is not particularly limited insofar as it has a columnar (square cup) shape.

A material for forming the well array is not particularly limited and can be selected as needed depending on the purpose. Examples include known glass materials, semiconductor materials, and resin materials.

A method of forming the well array is also not particularly limited and can be selected as needed depending on the purpose. Examples include known methods such as a method of pattern drawing by lithography and then etching to form the well array, and a method of forming the well array by injection molding or imprint with a mold having a shape of the well.

For example, the processing limit of a method of forming the well array by lithography and reactive ion etching with silicon as a formation material is about 0.1 nm and thus the well array can be formed with high resolution.

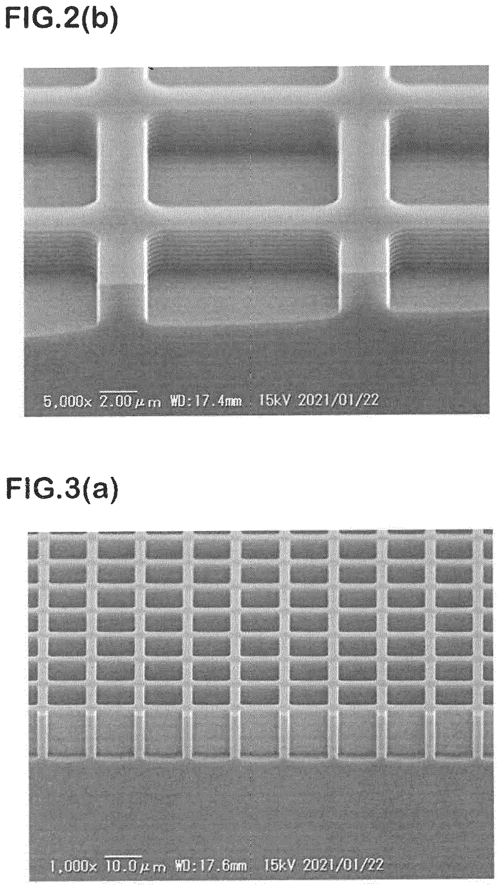

Preparation Example 1 of the well array is shown in FIGS. 2(a) and (b). FIG. 2(a) shows an electron microscope image in which Preparation Example 1 of the well array has been imaged and FIG. 2(b) is a partially enlarged photograph of FIG. 2(a).

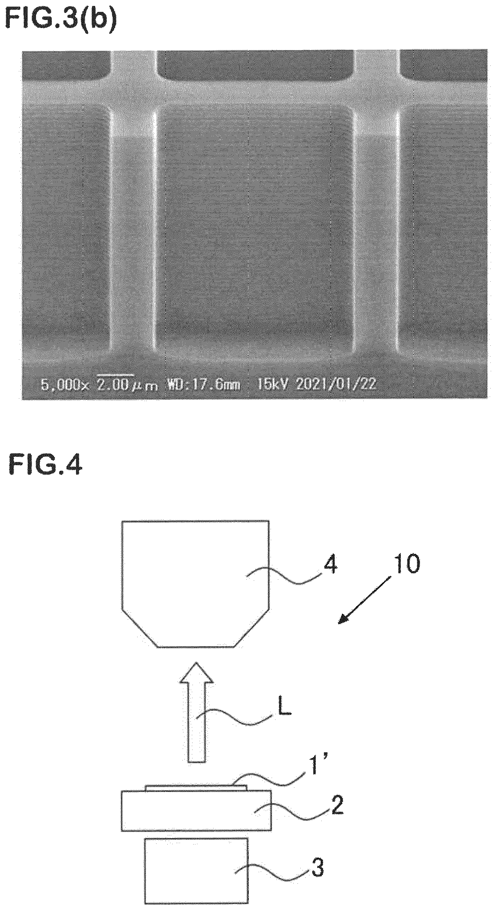

Also, Preparation Example 2 of the well array is shown in FIGS. 3(a) and (b). FIG. 3(a) shows an electron microscope image in which Preparation Example 2 of the well array has been imaged and FIG. 3(b) is a partially enlarged photograph of FIG. 3(a).

Preparation Examples 1 and 2 are each obtained by lithography and reactive ion etching with silicon as a formation material.

The well array of Preparation Example 1 relates to a preparation example of a shallow type in which a well 1 is formed while setting the opening shape to a square with 10 $\mu$m on each side, a depth D to 3 $\mu$m, and a side wall thickness T to 3 $\mu$m.

The well array of Preparation Example 2 relates to a preparation example of a deep type in which a well 1 is formed while setting the opening shape to a square with 10 $\mu$m on each side, a depth D to 15 $\mu$m, and a side wall thickness T to 3 $\mu$m.

The well array of Preparation Example 1 is suitably used for the particles having a small particle size and the well array of Preparation Example 2 is preferably used for the particles having a large particle size.

<Color development detection step>

The color development detection step is to detect color development of the well by using a commercially available image pickup element.

In the color development detection step, a generally available image pickup element may be used without a particular problem. An image pickup element having at least 300,000 pixels may be used.

The aforesaid image pickup element constitutes a detection unit based on a known microscope or the like and used for the detection of color development of the well. In the color development detection of the well with the observation visual field of 5 mm$^2$, for example, the detection unit may be comprised of a 4$\times$ objective lens, while in the color development detection of the well with the observation visual field of 0.8 mm$^2$, for example, the detection unit may be comprised of a 10$\times$ objective lens.

Although the color development of the well is not particularly limited, it preferably occurs, after the previous particle storing step, due to a reaction between the biological substance in the trapped body and a color producing reagent causing color development of the well.

The color producing reagent is not particularly limited and examples include a color producing reagent used in a known biological substance detection method such as an ELISA method and an immunoassay method. Specific examples include a color development substance that adsorbs to the biological substance and causes color development of the well, a reagent that forms a fluorescent substance by an enzyme reaction with a protein in the biological substance, a reagent that forms a chemiluminescent substance by an enzyme reaction with a protein in the biological substance, and a labeled substance having a recognition site that specifically recognizes the biological substance.

The color producing reagent may be added to the well before and after the particle storing step or may be added during preparation of the test liquid in the test liquid preparation step.

The term "color development" as used in this specification means a state in which light different in at least one of spectrum and intensity between the well containing the biological substance and the well not containing the biological substance can be detected. The concept "color development" includes, in addition to a change in coloration from the well not containing the biological substance to the well containing the biological substance, at least one of a change in emission spectrum and a change in emission intensity from the well not containing the biological substance to the well containing the biological substance. The term "color development" as used in this specification may be replaced with "color development or light emission".

The color development substance that adsorbs to the biological substance to cause color development of the well is not particularly limited and it is for example an aggregation induced emission (AIE) substance. Examples of the aggregation induced emission substance include compounds producing an AIE effect as described in Japanese Patent Laid-Open No. 2010-112777.

Examples of the reagent that causes an enzyme reaction with a protein in the biological substance and thereby produces a fluorescent substance include 4-methylumbelliferone-containing derivatives such as (4-methylumbelliferyl)-$\alpha$-D-N-acetylneuraminic acid that enzymatically reacts with neuraminidase in an influenza virus and thereby produces 4-methylumbelliferone which is a fluorescent substance, fluorescein-containing derivatives, resorufin-containing derivatives, and rhodamine-containing derivatives.

Examples of the reagent that produces a chemiluminescent substance by an enzyme reaction with a protein include luciferin that causes the protein to emit light as luciferase.

Examples of the labeled substance include a labeled enzyme or a labeled fluorescent dye in which an antibody recognizing the biological substance is labeled by an enzyme or a fluorescent dye.

An example of a method of performing the biological substance detection method using the magnetic particles as the particles will be described with reference to drawings.

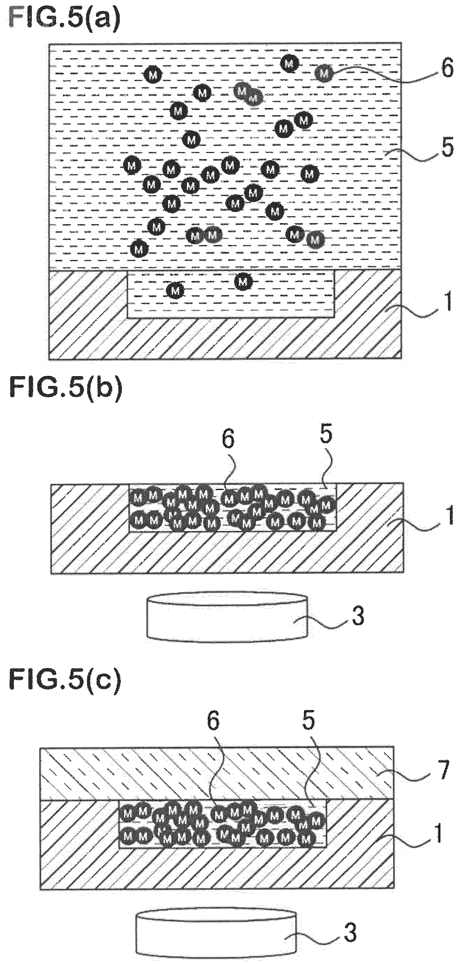

FIG. 4 shows an embodiment of a detection device to be used for the biological substance detection method. FIGS. 5(a) to (c) show detection manners of the biological substance.

As shown in FIG. 4, a detection device 10 is comprised of a detection chip 2 in which a well array 1' having a plurality of the wells 1 is placed, a detection unit 4, and a magnetic field application unit 3. The detection chip 2 is not particularly limited and it has a constitution similar to that of a known detection chip used for biological substance observation. The magnetic field application unit 3 is not particularly limited and it is comprised of a permanent magnet, an electromagnet or the like.

First, a test liquid 5 is prepared so as to have a concentration of magnetic particles 6 of at least $5 \times 10^7$ particles/mL and then the test liquid 5 is stirred. For example, after the magnetic particles 6 trap the biological substance to form the trapped body, the test liquid 5 is sent onto the well array 1' (refer to FIG. 5(a)). The number of the magnetic particles 6 shown in the drawings is simplified so as not to complicate the drawings.

Next, after the test liquid is sent, the magnetic particles 6 are attracted into the well 1 by a magnetic field applied from the magnetic field application unit 3 and the plurality of the magnetic particles 6 including the trapped body are stored in the well 1 (refer to FIG. 5(b)). At this time, the excess test liquid 5 is sent outside of the well array 1'. When the magnetic particles 6 are replaced by the plastic particles, metal particles, or ceramic particles, the plastic particles and others are precipitated by their own weight and stored in the well 1.

Next, the color producing reagent is added in the well 1 and the well 1 is covered with a transparent glass plate 7 to prevent the magnetic particle 6 and the color producing reagent from dissipating from the well 1 (refer to FIG. 5(c)). Alternatively, a hydrophobic solvent may be dropped on the upper portion of the well 1 to seal the upper portion of the well 1 and then the transparent glass plate 7 may be placed thereon. The color producing reagent may be added to the test liquid 5 in advance just before the test liquid 5 is sent onto the well array 1'.

When a reaction between the biological substance trapped in the trapped body and the color producing reagent proceeds, color development due to this reaction occurs. This color development is detected at the detection unit 4 as color development L of the well 1 (refer to FIG. 4).

When the color producing reagent produces fluorescence in the well 1, the aforesaid reaction is followed by emission of an excited light from an arbitrary light source to cause fluorescence and this color development is detected at the detection unit 4 as color development L of the well 1.

According to the biological substance detection method described above, all the particles are stored in the well to keep a detection sensitivity, and, in addition, by using the test liquid prepared to have a high particle concentration, a reaction time necessary for trapping the biological substance is reduced and thereby high-speed detection of the biological substance can be achieved.

(Well array)

The well array of the present invention is used for the biological substance detection method of the present invention and it can store, in one of the wells, a plurality of the particles including the trapped body while storing the number of the particles to at least the minimum average storage number $N_{min}$ represented by the following formulas (1) and (2).

According to the well array, all the particles are stored in the well to keep a detection sensitivity, and, in addition, by using the test liquid prepared to have a high particle concentration, a reaction time necessary for trapping the biological substance is reduced and thereby high-speed detection of the biological substance can be achieved.

[Mathematical Formula 5]

$$N_{min} = C \times V_L \times \frac{P_{image}}{P_{total}} \quad (1)$$

$$N_{min} \geqq 8 \quad (2)$$

wherein, in the formula (1), C represents the concentration of the particles in the test liquid and it is at least $5 \times 10^7$ particles/mL; $V_L$ represents an amount of the test liquid and is at least 5 $\mu$L; $P_{total}$ represents the number of pixels of the image pickup element and is at least 300,000 pixels; and $P_{image}$ represents the number of pixels of a pixel group used for imaging one of the wells in the image pickup element and is at least 9 pixels ($3 \times 3$ pixels).

The well array can be formed by applying the above matters described in the biological substance detection method and an overlapping description is omitted.

(Detection device)

The detection device of the present invention has the detection chip on which the well array of the present invention is placed and the detection unit having the image pickup element with at least 300,000 pixels.

According to the detection device, all the particles are stored in the well to keep a detection sensitivity, and, in addition, by using the test liquid prepared to have a high particle concentration, a reaction time necessary for trapping the biological substance is reduced and thereby high-speed detection of the biological substance can be achieved.

The detection device can be formed by applying the above matters described in the biological substance detection method and an overlapping description is omitted.

Examples

In order to confirm the validity of the biological substance detection method of the present invention, the following detection test was conducted with an influenza virus (biological substance) as a detection target.

In the detection test, a well array was used in which $155 \times 155$ wells, total 24,025 wells, each well having a square opening shape with 10 $\mu$m on each side, a depth D of 5 $\mu$m, and a volume of 500 fL, were formed with a side wall thickness T of 3 $\mu$m on a silicon substrate. The well array was prepared by a known shape processing method using photolithography and dry etching.

As a particle for trapping influenza viruses, 1 $\mu$m diameter magnetic particles having a solid-phased anti-hemagglutinin antibody were used.

As a reagent for detecting the influenza viruses, (4-methylumbelliferyl)-$\alpha$-D-N-acetylneuraminic acid (MUNANA, (produced by Toronto Research Chemicals, M334200)), a reagent for producing a fluorescent substance by an enzyme reaction, was used.

As a camera for observing the well array, a CMOS camera with $2,048 \times 2,048$ pixels (4,194,304 pixels, $P_{total}$) (ORCA-Flash4.0 V3, manufactured by Hamamatsu Photonics K.K.) was used.

The following is the details of the detection method. First, 15 $\mu$L of a specimen liquid containing the influenza viruses was mixed with 15 $\mu$L of a magnetic particle liquid containing the magnetic particles at a concentration of $5 \times 10^8$ particles/mL, and the resulting mixture was left to stand at room temperature for 10 minutes to cause reaction therebetween so as to bind the influenza viruses to the magnetic particles.

Then, 30 $\mu$L of a MUNANA solution having a concentration of 1 mM was added and mixed with the reaction product. A 50 $\mu$L portion of the resulting mixture was collected and introduced into the well array. The concentration (C) of the magnetic particles in the mixture (the test liquid, $V_L$ = 50 $\mu$L) collected and introduced into the well array was $1.25 \times 10^8$ particles/mL.

Then, the magnetic particles were drawn into the well array by using a magnet, and the well array was sealed with a fluorine oil and thereafter covered with a cover glass. After that, the well array was observed with a fluorescence microscope (BXFM, manufactured by OLYMPUS CORPORATION).

The observation was performed under the conditions where a $4 \times$ objective lens was used and the wells were each observed by 64 pixels ($8 \times 8$ pixels, $P_{image}$) of the CMOS camera.

In the well array, a fluorescent substance 4-methylumbelliferone was formed by the enzyme reaction between the influenza viruses and the MUNANA introduced into the wells. In fact, an increase of luminescence due to the enzyme reaction was observed in the wells containing the influenza viruses and the wells were detected as luminescent wells.

The number of the luminescent wells for 20 minutes of an enzyme reaction time was counted and it was normalized with the number of the wells used for the observation. The value thus obtained was used as a measurement value corresponding to the concentration of the influenza viruses.

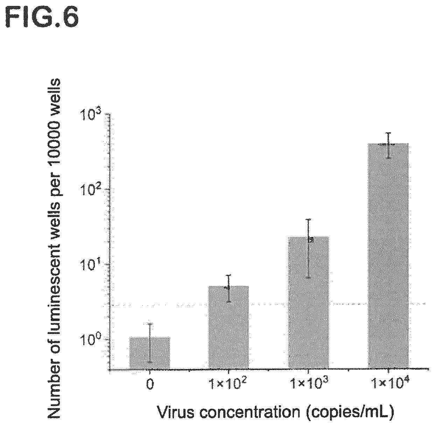

The results of the present test are shown in FIG. 6.

As shown in FIG. 6, the measurement value of the number of the luminescent wells correlates to the concentration of the influenza viruses and an increasing tendency with an increase in the concentration of the influenza viruses is observed. This means that the presence of the influenza viruses and its concentration can be detected from the number of the luminescent wells.

It is to be noted that the lower detection limit determined by $\mu + 3.3\sigma$ (shown by a dotted line in FIG. 6) wherein $\mu$ is an average number of the luminescent wells measured using a sample not containing the influenza viruses and $\sigma$ is a standard deviation is $1 \times 10^2$ copies/mL.

The trapping time by the magnetic particles was 10 minutes and the measurement time including the enzyme reaction time (20 minutes) was about 30 minutes.

The lower detection limit is remarkably small and the measurement time is remarkably short so that this method has performance exceeding that of the PCR method widely used now as a high-sensitivity virus detection method.

In the present test, the value $N_{min}$ determined from the formula (1) is about 95 and the average storage number of the magnetic particles in the wells is almost 260, which satisfy the condition of $N_{min}$ specified by the formula (2). In short, according to the present invention, high-speed and high-sensitivity biological substance detection is realized.

Description of Reference Numerals

1:      well
1':      well array

2: detection chip
3: magnetic field application unit
4: detection unit
5: test liquid
6: magnetic particles
7: transparent glass plate
10: detection device
L: color development

## Claims

1. A biological substance detection method using a well array formed by defining a plurality of wells adjacent to each other with a side wall stood on a substrate and particles capable of trapping a biological substance and detecting the biological substance in a test liquid based on color development detection of the wells, comprising:

   a test liquid preparation step for preparing the test liquid containing the particles at a concentration of at least $5 \times 10^7$ particles/mL,
   a biological substance trapping step for allowing the particles to trap the biological substance to form a trapped body,
   a particle storing step for sending the test liquid onto the well array to store a plurality of the particles including the trapped body in the wells while storing the number of the particles to be stored into one of the wells to at least a minimum average storage number $N_{min}$ represented by the following formulas (1) and (2), and
   a color development detection step for detecting color development of the wells with an image pickup element having at least 300,000 pixels.

   [Mathematical Formula 1]

   $$N_{min} = C \times V_L \times \frac{P_{image}}{P_{total}} \quad (1)$$

   $$N_{min} \geqq 8 \quad (2)$$

   wherein, in the formula (1), C represents the concentration of the particles in the test liquid and is at least $5 \times 10^7$ particles/mL; $V_L$ represents the amount of the test liquid and is at least $5 \mu L$; $P_{total}$ represents the number of pixels of the image pickup element and is at least 300,000 pixels; and $P_{image}$ represents the number of pixels of a pixel group used for imaging one of the wells in the image pickup element and is at least 9 pixels ($3 \times 3$ pixels).

2. The biological substance detection method according to Claim 1, wherein the particles are magnetic particles having a diameter of 0.1 $\mu m$ to 9 $\mu m$.

3. The biological substance detection method according to Claim 1 or 2, wherein the test liquid preparation step is to prepare the test liquid while adjusting the concentration of the particles to $5 \times 10^7$ particles/mL to $5 \times 10^9$ particles/mL.

4. The biological substance detection method according to any of Claims 1 to 3, wherein the wells each have a volume of 2 fL to 100 pL.

5. The biological substance detection method according to any of Claims 1 to 4, wherein the color development detection step is carried out by fixing an observation visual field of the image pickup element to have a size including a well formation area which is an area of the substrate in a well formation region.

6. The biological substance detection method according to any of Claims 1 to 5, wherein color development of the wells in the color development detection step is due to a reaction between the biological substance of the trapped body and a color producing reagent causing the color development of the wells.

7. A well array for use in the biological substance detection method as claimed in any of Claims 1 to 6,

which can store a plurality of the particles including the trapped body in one of the wells while storing the number of the particles to at least a minimum average storage number $N_{min}$ represented by the following formulas (1) and (2):

[Mathematical Formula 2]

$$N_{min} = C \times V_L \times \frac{P_{image}}{P_{total}} \qquad (1)$$

$$N_{min} \geqq 8 \qquad (2)$$

wherein, in the formula (1), C represents the concentration of the particles in the test liquid and is at least $5 \times 10^7$ particles/mL; $V_L$ represents the amount of the test liquid and is at least 50 $\mu$L; $P_{total}$ represents the number of pixels of the image pickup element and is at least 300,000 pixels; and $P_{image}$ represents the number of pixels of a pixel group used for imaging one of the wells in the image pickup element and is at least 9 pixels ($3 \times 3$ pixels).

8. A detection device, comprising:

a detection chip having the well array as claimed in Claim 7, and
a detection unit having an image pickup element having a pixel number of at least 300,000 pixels.

# FIG.1

# FIG.2(a)

FIG.2(b)

5,000x 2.00 μm  WD:17.4mm  15kV 2021/01/22

FIG.3(a)

1,000x 10.0 μm  WD:17.6mm  15kV 2021/01/22

## FIG.3(b)

5,000x 2.00 μm WD:17.6mm 15kV 2021/01/22

## FIG.4

FIG.5(a)

FIG.5(b)

FIG.5(c)

FIG.6

## INTERNATIONAL SEARCH REPORT

| International application No. |
| --- |
| **PCT/JP2022/009369** |

**A. CLASSIFICATION OF SUBJECT MATTER**

*G01N 21/64*(2006.01)i; *C12M 1/34*(2006.01)i; *C12Q 1/70*(2006.01)i; *G01N 21/03*(2006.01)i; *G01N 21/78*(2006.01)i; *G01N 33/483*(2006.01)i

FI: G01N21/64 Z; C12M1/34 B; G01N21/03 Z; G01N21/78 C; G01N33/483 C; C12Q1/70

According to International Patent Classification (IPC) or to both national classification and IPC

**B. FIELDS SEARCHED**

Minimum documentation searched (classification system followed by classification symbols)

G01N21/00-21/83; C12M1/34; C12Q1/04; C12Q1/70; G01N33/48-33/98; G01N37/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2018/181488 A1 (JAPAN SCIENCE & TECH AGENCY) 04 October 2018 (2018-10-04) | 1-8 |
| A | WO 2016/006208 A1 (JAPAN SCIENCE & TECH AGENCY) 14 January 2016 (2016-01-14) | 1-8 |
| A | JP 5551798 B2 (QUANTERIX CORPORATION) 16 July 2014 (2014-07-16) | 1-8 |
| A | MINAGAWA, Yoshihiro et al. Mobile imaging platform for digtal influenza virus counting. Lab on a Chip. 21 August 2019, vol. 19/16, 2678-2687 abstract, fig. 1-3 | 1-8 |
| A | LEIRS, K. et al. Bioassay Development for Ultrasensitive Detection of Influenza A Nucleoprotein Using Digital ELISA. Analytical Chemistry. 04 August 2016, vol. 88/17, 8450-8458, https://doi.org/10.1021/acs.analchem.6b00502 abstract, fig. 3 | 1-8 |
| A | TABATA, K. V. et al. Antibody-free digital influenza virus counting based on neuraminidase activity. Scientific Reports. 31 January 2019, vol. 9/1067, 1-13, https://www.nature.com/articles/s41598-018-37994-6.pdf abstract, fig. 1-2 | 1-8 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **12 May 2022** | **24 May 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/009369**

| Patent document cited in search report | | | Publication date (day/month/year) | Patent family member(s) | | | Publication date (day/month/year) |
|---|---|---|---|---|---|---|---|
| WO | 2018/181488 | A1 | 04 October 2018 | US | 2021/0278323 | A1 | |
| | | | | EP | 3605110 | A1 | |
| | | | | CN | 110476067 | A | |
| WO | 2016/006208 | A1 | 14 January 2016 | US | 2015/0087547 | A1 | |
| | | | | US | 2017/0176430 | A1 | |
| | | | | EP | 3168624 | A1 | |
| | | | | CA | 2953666 | A1 | |
| | | | | KR | 10-2017-0027716 | A | |
| | | | | CN | 106662599 | A | |
| JP | 5551798 | B2 | 16 July 2014 | WO | 2011/109364 | A2 | |
| | | | | US | 2011/0212848 | A1 | |
| | | | | US | 2012/0277114 | A1 | |
| | | | | US | 2012/0289428 | A1 | |
| | | | | US | 2018/0017552 | A1 | |
| | | | | US | 2019/0302109 | A1 | |
| | | | | US | 2020/0393457 | A1 | |
| | | | | EP | 2542891 | A2 | |
| | | | | EP | 2995955 | A1 | |
| | | | | EP | 3330709 | A1 | |
| | | | | CA | 2791654 | A1 | |
| | | | | CN | 102884431 | A | |
| | | | | CN | 104820092 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20166208 A
- JP 2018091737 A
- WO 2012121310 A
- JP 3886161 B
- JP 5551798 B
- JP 5363663 B
- JP 2010112777 A

### Non-patent literature cited in the description

- **DAVID M RISSIN et al.** *Nature Biotechnology,* 2010, vol. 28 (6), 595
- **ELENA PEREZ-RUIZ et al.** *Analytica Chimica Acta,* 2018, vol. 1015, 74
- **SOO HYEON KIM et al.** *Lab on a Chip,* 2012, vol. 12, 4986
- **DAVID M RISSIN et al.** *Analytical Chemistry,* 2011, vol. 83, 2279
- **STEPHANIE M. SCHUBERT et al.** *Analytical Chemistry,* 2016, vol. 88, 2952
- **KAREN LEIRS et al.** *Analytical Chemistry,* 2016, vol. 88, 8450